## Europäisches Patentamt

## European Patent Office

## Office européen des brevets

(19)

(11) Numéro de publication: **0 114 769**
**B1**

(12) **FASCICULE DE BREVET EUROPEEN**

(45) Date de publication du fascicule du brevet:
08.10.86

(51) Int. Cl.⁴: **C 12 P 5/02**

(21) Numéro de dépôt: **84400097.6**

(22) Date de dépôt: **18.01.84**

(54) **Procédé de production de méthane par fermentation anaérobie.**

(30) Priorité: **20.01.83 FR 8300824**

(43) Date de publication de la demande:
**01.08.84 Bulletin 84/31**

(45) Mention de la délivrance du brevet:
**08.10.86 Bulletin 86/41**

(84) Etats contractants désignés:
**BE DE GB IT NL**

(56) Documents cités:
FR - A - 879 390

BIOMASS, vol. 2, 1982, pages 43-55, Applied Science
Publishers Ltd., GB D.A. STAFFORD: "The effects of
mixing and volatile fatty acid concentrations on
anaerobic digester performance"
WATER RESEARCH, vol. 10, 1976, pages 9-18,
Pergamon Press, GB CHENG-NAN WENG et al.:
"Biochemical mechanisms in the methane fermentation
of glutamic and oleic acids"
ARGONNE NATL. LAB., ENERGY ENVIRON. SYST. DIV.,
(TECH. REP.) vol. 2, 1980, PROC. U.S. DEP. ENERGY
OPTIM. WATER WASTEWATER MANAGE. MUNIC. IND.
APPL. CONF. 1979, pages 409-16 F. KREMER et al.:
"Acclimation of anaerobic digesters to industrial
wastes with the addition metals without loss of

(73) Titulaire: **SOCIETE NATIONALE ELF AQUITAINE, Tour
Aquitaine, F-92400 Courbevoie (FR)**

(72) Inventeur: **Finck, Jean-Daniel, 17 Rue Jean Mermoz,
F-31520 Ramonville St Agne (FR)**
Inventeur: **Goma, Gérard, 20, rue Salas,
F-31520 Ramonville St Agne (FR)**

(74) Mandataire: **Ohresser, François, Société Nationale Elf
Aquitaine Division Proprieté Industrielle Tour Elf,
F-92078 Paris La Défense Cédex 45 (FR)**

(56) Documents cités: (suite)
methane production"
CHEMICAL ABSTRACTS, vol. 63, 1965, colonne 1578g,
Columbus, Ohio, US G. BURACZEWSKI: "Methane
fermentation of sewage sludge. I. The influence of
physical and chemical factors on the development of
methane bacteria and the course of fermentation"
CHEMICAL ABSTRACTS, vol. 95, 1981, page 154, no.
100483q, Columbus, Ohio, US HANAKI KEISUKE et al.:
"Mechanism of inhibition caused by long-chain fatty
acids in anaerobic digestion process"
CHEMICAL ABSTRACTS, vol. 98, 1983, page 336, no.
95087u, Columbus, Ohio, US G. HANSSON: "Methane
production from glucose and fatty acids at 55-85C:
adaption of cultures and effects of pCO2"

**Description**

La présente invention concerne un procédé de production de méthane par fermentation anaérobie de substrats organiques.

Les procédés de production de biogaz sont connus depuis de nombreuses années et les conditions opératoires de la fermentation ont fait l'objet de nombreux travaux. L'un des inconvénients majeur des procédés de ce type est le faible rendement des fermentations dû en partie à la faible vitesse de certaines réactions bactériennes. Cette faible vitesse amène très souvent les utilisateurs à mettre en œuvre des réacteurs de grand volume pour augmenter ainsi le temps de séjour du substrat.

L'invention a pour but de pallier des inconvénients et de permettre de mettre en œuvre un procédé de production de biogaz ayant des rendements améliorés.

L'invention a pour objet un procédé de production de méthane par fermentation anaérobie de substrats organiques, mis en œuvre dans les conditions habituelles de fermentation caractérisé, en ce qu'on ajoute au milieu de fermentation au moins un acide gras insaturé, ou un ester de cet acide, à une concentration comprise entre 0,001 et 0,35 millimoles par litre de milieu de culture et telle que, pour l'acide gras considéré, le rendement en biogaz est au moins égal à celui obtenu dans les mêmes conditions en l'absence dudit acide gras.

On utilisera dans le procédé de l'invention un acide gras, seul ou en mélange, ayant par molécule, de 18 à 22 atomes de carbone par molécule et au moins deux insaturations oléfiniques.

L'influence des acides gras insaturés comportant 18 atomes de carbone sur la production de méthane a déjà été étudiée et a fait l'objet d'une publication de MM. D.I. DEMEYER et H.K. HENDERICKS dans «Biochimica et Biphysica Acta, 137 (1967) pp 484-497. Il ressort clairement des conclusions de cette étude que ce type d'acides est un inhibiteur de la production de méthane.

Par ailleurs, D.A. STAFFORD dans «BIOMASS Vol. 2, 1982 pages 43-45» traite de l'effet de la concentration en acides gras volatils sur le rendement des digestions anaérobies. Dans ce cadre, il mentionne que l'acide oléique, précurseur par hydrolyse des acides gras volatils, peut avoir, lorsqu'il est utilisé en quantité importante, un effet favorable sur la production de gaz.

Or il à été découvert, avec surprise, que les acides gras ou les dérivés de ces acides tels que les esters, peuvent être dans certaines conditions des activateurs puissants de la production de méthane par fermentation anaérobie de substrats organiques.

Il est essentiel pour cela que l'acide gras soit ajouté au milieu de culture à des très faible concentrations. Ces concentrations seront généralement comprises entre 0,001 et 0,35 millimoles par litre de milieu de culture. Les concentrations optimales varieront, dans le domaine défini ci-dessus, selon l'acide gras mis en oeuvre dans le procédé. Il sera nécessaire pour chaque acide gras mis en oeuvre de vérifier que, pour la concentration choisie, le rendement en biogaz est au moins égal à celui obtenu avec une culture témoin sans acide gras ($R \geq 1$), R étant le rapport des volumes de biogaz produits expérimentalement (Vex) et en essai témoin (Vt).

Naturellement l'acide gras ou son dérivé devra être soluble dans le milieu de culture; s'il ne l'est pas il sera alors préférable de l'émulsifier préalablement à l'addition. Parmi les acides gras ou leurs dérivés que l'on peut utiliser de préférence dans le procédé de l'invention on peut citer à titre d'exemple:

— l'acide linoléique
— l'acide linolénique
— l'acide arachidonique
— l'acide clupanodonique
— les esters de ces acides
— les mélanges de ces acides tel que l'huile de lin.

Lorsqu'on utilisera l'acide linoléique ou l'huile de lin comme acide gras il sera particulièrement avantageux de l'ajouter à une concentration comprise entre 0,10 et 0,25 mM par litre (mM étant l'abréviation de millimole).

Dans le cas où l'on choisit l'acide linolénique ou ses dérivés il est essentiel de l'utiliser à une concentration plus faible comprise entre 0,001 et 0,1 mm par litre. Au delà de cette concentration le rendement en biogaz chute considérablement ($R < 1$).

Le procédé de l'invention est illustré par les exemples 1 à 32 décrits ci-dessous.

*Exemples 1 à 29*

L'étude de la production de biogaz par fermentation d'un substrat constitué de fumier de bovin ramené à 10% de matière sèche a été menée en discontinu avec divers acides gras à différentes concentrations, et ceci dans des conditions opératoires identiques. La fermentation est réalisée à 35°C à un pH moyen initial de 7,4 dans les bouteilles de verre non agitées.

Les fermentations sont menées en général durant 30 jours dans certains cas, limitées à 8 jours. Le critère de comparaison du rendement est la production de biogaz par rapport à celle d'un essai témoin (ex 1), sans acide, étant précisé que la teneur en méthane du biogaz est égale à 55% et identique dans toutes ces expériences.

Les conditions opératoires précises et les résultats obtenus sont indiqués dans le tableau I ci-dessous.

TABLEAU 1

| Ex | Produit ajouté | Nature de l'acide gras[1] | Concentration mM/l | Production biogaz après 8 jours | Production biogaz après 30 jours |
|---|---|---|---|---|---|
| 1 | Témoin | — | — | 100 | 100 |
| 2 | Acide caprique | $C_{10}$ saturé | 0,35 | 100,7 | — |
| 3 | Acide myristoléique | $C_{14}$ I | 0,13 | 96 | — |
| 4 | Acide stéarique | $C_{18}$ saturé | 0,09 | 98 | |
| 5 | | | 0,21 | 96,5 | — |
| 6 | Acide oléique | $C_{18}$ I | 0,07 | 110 | 106 |
| 7 | | | 0,14 | 110 | 103,5 |
| 8 | | | 0,21 | 102,5 | 102,5 |
| 9 | | | 0,53 | 105 | 103,5 |
| 10 | Acide linoléique | $C_{18}$ II | 0,07 | 142 | 127,5 |
| 11 | | | 0,14 | 206 | 209 |
| 12 | | | 0,21 | 237 | 222 |
| 13 | | | 0,53 | 37,7 | 23 |
| 14 | Acide linolénique | $C_{18}$ III | 0,017 | 162 | 141 |
| 15 | | | 0,035 | 162 | 160,5 |
| 16 | | | 0,09 | 227 | 217 |
| 17 | | | 0,18 | 47,1 | 29 |
| 18 | Acide ricinoléique | $C_{18}$ OH | 0,2 | 91 | — |
| 19 | Acide arachidonique | $C_{20}$ IV | 0,08 | 111 | — |
| 20 | Acide clupanodonique | $C_{22}$ V | 0,06 | 125 | — |
| 21 | Huile de lin | $C_{18}$ I 18% | 0,07 | 107,5 | 133 |
| 22 | | $C_{18}$ II 19% | 0,14 | 88 | 207 |
| 23 | | $C_{18}$ III 51% | 0,21 | 168 | 272,5 |
| 24 | | autres 12% | 0,53 | 22 | 10 |
| 25 | Ester d'acide gras insaturé | Monolinolénate d'oxypropylène-glycol | 0,027 | — | — |
| 26 | | | 0,054 | — | — |
| 27 | | | 0,13 | 117 | 114 |
| 28 | | | 0,37 | 100 | 110 |
| 29 | | | 0,52 | 37,5 | 8 |

(1) l'indice en chiffre romain précise le nombre de double liaison oléfinique de la molécule ($C_{18}$ III = Acide de 18 atomes de carbone avec trois insaturations oléfiniques).

L'analyse du tableau 1, montre que:

— les acides gras saturés sont totalement inactifs

— les acides gras mono-ethyléniques sont faiblement actifs (exemples 6 à 9)

— les acides gras di-éthyléniques et triéthyléniques sont particulièrement actifs (exemples 10 à 12 et 14 à 16 respectivement); ils deviennent inactifs et même inhibiteurs dès que leurs concentration est trop importante (exemple 13 et 17). Il est à remarquer que l'acide linolénique est très actif à faible concentration et devient inhibiteur à une concentration peu élevée.

— l'huile de lin est active et cette activité augmente au cours du temps (exemples 21 à 23)

— les gains en rendement sont extrèmement importants ceux ci dépassant 200%.

*Exemples 30 à 32*

Des expériences comparatives ont été menées en pilote continu de 5m3 dans les conditions expérimentales suivantes, l'alimentation du réacteur s'effectuant 5 jours par semaine:

| | |
|---|---|
| Température | 35°C |
| pH | 6,8 - 7,3 |
| Taux de charge moyen | 6,7 Kg Solides volatils/m3. J |
| Temps de résidence théorique moyen | 17,4 j |
| Nature moyenne de la charge: | |
| — matière sèche totale | 13,86 % poids |
| — solides volatils | 80,7 % MST |
| — cellulose | 17,15 % MST |
| — hémicelluloses | 18,2 % MST |
| — lignine | 14,1 % MST |
| — azote total | 1,92 % MST |

Les résultats obtenus sans acide gras (ex 30), avec 0,027mM/l du monolinolénate des exemples 25 à 39 (ex 31) et 0,054 mM de ce monolinolénate (ex 32) sont résumés dans le tableau (2) ci-dessous.

TABLEAU 2

| Exemples | 30 | 31 | 32 |
|---|---|---|---|
| Productivité biogaz brut (m3/m3 .j) | 1,76 | 2,50 | 2,31 |
| Teneur en CH$_4$ (%) | 55 | 55 | 55 |
| Rendement par rapport au témoin (%) | 100 | 142 | 131,5 |
| Teneur en monolinolénate (mM/l) | 0 | 0,027 | 0,054 |

Le procédé de l'invention peut être utilisé pour traiter tout substrat fermentiscible convenable et en particulier les substrats riches en cellulose et/ou ligno cellulose.

Le substrat peut être d'origine animale, par exemple des lisiers, des fumiers ou des effluents d'abattoirs; il peut être également d'origine végétale par exemple des déchêts de cultures telle que la luzerne, le topinambour, la canne à sucre, le maïs etc..., enfin il peut être d'origine industrielle et constitué par exemple d'effluents de laiteries, de conserveries, de l'industrie papetière etc...

## Revendications

1. Procédé perfectionné de production de méthane par fermentation anaérobie de substrats organiques, mit en œuvre dans les conditions habituelles de fermentations, caractérisé en ce qu'on ajoute au milieu de fermentation au moins un acide gras insaturé ou un ester de cet acide, ledit acide gras comportant de 18 à 22 atomes de carbone et au moins deux insaturations oléfiniques par molécule, à une concentration comprise entre 0,001 et 0,35 millimoles par litre de milieu de fermentation telle que, pour l'acide gras considéré, le rendement en biogaz R soit au moins égal à 1.

2. Procédé selon la revendication 1 caractérisé en ce que l'acide gras est l'acide linolénique.

3. Procédé selon la revendication 1 caractérisé en ce que l'acide gras est l'acide linoléique.

4. Procédé selon la revendication 1 caractérisé en ce que l'acide gras est constitué par de l'huile de lin.

5. Procédé selon la revendication 2 caractérisé en ce que l'acide gras est un ester de l'acide linolénique.

6. Procédé selon l'une des revendications 2 et 5 caractérisé en ce que la concentration en acide gras est comprise entre 0,001 et 0,10 mM/l.

## Patentansprüche

1. Verbessertes Verfahren für die Herstellung von Methan durch anaerobe Fermentierung organischer Substrate, das unter den üblichen Fermentierungsbedingungen durchgeführt wird, dadurch gekennzeichnet, dass man dem Fermentationsmedium mindestens eine ungesättigte Fettsäure oder einen Ester dieser Säure zugibt, wobei die Fettsäure 18 bis 22 Kohlenstroffatome aufweist und wenigstens zweifach olefinisch ungesättigt pro Molekül ist, wobei die Konzentration des Fermentierungsmediums zwischen 0,001 und 0,35 Millimol/Liter liegt, so dass die Ausbeute an Biogas R für die entsprechende Fettsäure wenigstens gleich 1 ist.

2. Verfahren nach Anspruch 1, dadurch gekennzeichnet, dass die Fettsäure Linolensäure ist.

3. Verfahren nach Anspruch 1, dadurch gekennzeichnet, dass die Fettsäure Linolsäure ist.

4. Verfahren nach Anspruch 1, dadurch gekennzeichnet, dass die Fettsäure Leinöl ist.

5. Verfahren nach Anspruch 2, dadurch gekennzeichnet, dass die Fettsäure ein Ester der Linolensäure ist.

6. Verfahren nach einem der Ansprüche 2 und 5, dadurch gekennzeichnet, dass die Konzentration der Fettsäure zwischen 0,001 und 0,10 mM/l liegt.

## Claims

1. Improved method of producing methane by anaerobic fermentation or organic substrates, employed in conventional fermentation conditions, characterised in that to the fermentation medium is added at least one unsaturated fatty acid or an ester of such acid, the said fatty acid comprising 18 to 22 carbon atoms and at least two olefin insaturations per molecule, at a concentration comprised between 0,001 and 0,35 millimoles per litre of fermentation medium, such that, for the fatty acid in question, the biogas R output is at least equal to 1.

2. Method according to Claim 1, characterised in that the fatty acid is linolenic acid.

3. Method according to Claim 1, characterised in that the fatty acid is linoleic acid.

4. Method according to Claim 1, characterised in that the fatty acid is constituted by linseed oil.

5. Method according to Claim 2, characterised in that the fatty acid is an ester of linolenic acid.

6. Method according to one of Claims 2 and 5, characterised in that the concentration of fatty acid is between 0.001 and 0.10 mM/l.